(19) Europäisches Patentamt
European Patent Office
Office européen des brevets

(11) Veröffentlichungsnummer: **0 317 911 B1**

(12) **EUROPÄISCHE PATENTSCHRIFT**

(45) Veröffentlichungstag der Patentschrift: **09.06.93**

(21) Anmeldenummer: **88119194.4**

(22) Anmeldetag: **18.11.88**

(51) Int. Cl.⁵: **C07C 45/58**, C07C 45/52, C07C 45/51, C07C 45/54, C07C 47/228, C07C 47/24, C07C 47/277

# (54) Verfahren zur Herstellung von Phenylacetaldehyden.

(30) Priorität: **27.11.87 DE 3740270**
**21.07.88 DE 3824725**

(43) Veröffentlichungstag der Anmeldung:
**31.05.89 Patentblatt 89/22**

(45) Bekanntmachung des Hinweises auf die Patenterteilung:
**09.06.93 Patentblatt 93/23**

(84) Benannte Vertragsstaaten:
**AT BE CH DE FR GB IT LI NL**

(56) Entgegenhaltungen:
**EP-A- 0 228 675**
**EP-A- 0 262 532**

**TETRAHEDRON LETTERS, Band 29, Nr. 12, 1988, Seiten 1471-1472; G. PARARATTO et al.: "A highly selective method for the synthesis of phenylacetaldehyde"**

**CHEMICAL ABSTRACTS, Band 105, Nr. 21, November 1986, Seite 689, Spalte 1, Zusammenfassung Nr. 1906622, Columbus, Ohio, US; & JP-A-61 112 040**

(73) Patentinhaber: **BASF Aktiengesellschaft**
**Carl-Bosch-Strasse 38**
**W-6700 Ludwigshafen(DE)**

(72) Erfinder: **Smuda, Hubert, Dr.**
**Rahmengasse 32**
**W-6900 Heidelberg(DE)**
Erfinder: **Hoelderich, Wolfgang, Dr.**
**Mannheimer Strasse 18 c**
**W-6710 Frankenthal(DE)**
Erfinder: **Goetz, Norbert, Dr.**
**Schoefferstrasse 25**
**W-6520 Worms(DE)**
Erfinder: **Recker, Hans-Gert, Dr.**
**2 Clove Blossom**
**Irvine, CA, 92714(US)**
Erfinder: **Hupfer, Leopold, Dr.**
**Waltershoehe 3**
**W-6701 Friedelsheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft ein Verfahren zur Herstellung von Phenylacetaldehyden aus Glycidsäureestern in Gegenwart von Zeolithen bzw. anderer Katalysatoren.

Es ist bekannt, Phenylacetaldehyde, durch Dehydrierung von Phenylethanolen bei Teilumsatz und einer verlustreichen Trennung von Ausgangsstoffen und Endprodukt herzustellen. Das Verfahren führt wegen der Thermolabilität der Phenylacetaldehyde bei der Fraktionierung zur Bildung von Selbstkondensationsprodukten und somit zu Ausbeuteverlusten. Auch eine Herstellung von halogenhaltigen Phenylacetaldehyden ist auf diesem Wege nicht möglich, da unter den Reaktionsbedingungen Halogenabspaltung erfolgt.

Weiterhin ist die Herstellung von Phenylacetaldehyden durch katalytische Umlagerung von Styroloxiden bekannt. In der Regel erzielt man ebenfalls nur einen Teilumsatz und erhält schwer abtrennbare Nebenprodukte.

In EP-A-100 117 wird die Reaktion von Styroloxid und von am aromatischen Kern alkyl- bzw. alkoxysubstituierten Styroloxiden an Titan-haltigen Zeolithen zu $\beta$-Phenylacetaldehyden in der flüssigen Phase bei 30 bis 100 °C beschrieben. Der Katalysator muß aufwendig aus teuren hochreinen Ausgangsstoffen wie Tetraalkylorthosilikat, Tetralkylorthotitanat und Tetrapropylammoniumhydroxid hergestellt werden. Auch andere Verfahren zur Umlagerung von Epoxiden zu Carbonylverbindungen sind bekannt. Beispielsweise Cyclododecanon wird an Pd- oder Rd-dotiertem $Al_2O_3$ aus Epoxycyclododecan erhalten (Neftekhimiya 16 (1976), 250-254). Es wird ausdrücklich darauf hingewiesen, daß Zeolithe für diese Reaktion ungeeignet sind. Auch der Einsatz von A-Zeolithen für die Umlagerung von Butylenoxid zu Butyraldehyd wird beschrieben (Hokkaido Daigaku Kogakubu Hokuku 67 (1973) 171-178). Die Selektivität (55 bis 72 %) läßt zu wünschen übrig. Die A-Zeolith-Katalysatoren sind nach einer Desaktivierung durch Koks schwer regenerierbar, da bei den hierfür notwendigen Temperaturen von etwa 500 °C die Kristallstruktur dieser Zeolithe zerstört wird.

Ferner ist aus der EP-A-228 675 ein Verfahren zur Herstellung von Phenylacetaldehyden aus Styroloxiden bzw. Phenylethylenglykolen bekannt, bei dem man Zeolithe des Pentasil-, Mordenit-, Erionit-, Chabazit- oder L-Typs verwendet und die Umsetzung bei 200 bis 500 °C , vorzugsweise bei 200 bis 400 °C und bei Normaldruck ausgeführt hat. Die Selektivitäten und Standzeiten der Katalysatoren, insbesondere bei Einsatz halogenierter Styroloxide bzw. Phenylethylenglykole lassen zu wünschen übrig.

Weiterhin ist bekannt, daß man Phenylacetaldehyde durch Umlagerung von Styrolglykol an Aluminiumsilikaten mit $SiO_2$:$Al_2O_3$ = 80:20 bis 93:7 gemischt mit z.B. Eisen-, Calcium- bzw. Magnesiumoxid oder an aktiviertem Ton in Suspension unter vermindertem Druck erhalten kann. Diesen beiden Verfahren ist gemeinsam, daß die Ausbeuten mit 50 bis 86 % noch verbesserungswürdig sind. Auch ist dieses Verfahren nicht flexibel, denn halogenierte Verbindungen werden nicht erhalten. Im Falle des Tons handelt es sich um ein natürliches Mineral, das je nach Provenience unterschiedliche Zusammensetzung und damit unterschiedliche katalytische Eigenschaften und Selektivität aufweist. Dies erschwert insbesondere ein kontinuierliches technisches Verfahren.

Aldehyde lassen sich auch in einer Rosenmund-Reduktion aus Carbonsäurechloriden gewinnen. Derartige Reaktionen gehen gut in der Flüssigphase mit Acrylsäurechloriden. Bei anderen Säurechloriden wie z.B. Aralkylcarbonsäurechloriden sind in der Regel niedrigere Ausbeuten gepaart mit Katalysatorvergiftung anzutreffen.

Der vorliegenden Erfindung lag die Aufgabe zugrunde, Verfahren zur Herstellung der Phenylacetaldehyde der Formel I aus leicht zugänglichen Ausgangsstoffen zu entwickeln, bei der in Gegenwart eines Katalysators, der einfach verfügbar sein soll und eine hohe Aktivität und hohe Selektivität bei langen Katalysatorstandzeiten haben soll, hohe Umsätze zu erreichen. Außerdem soll der Katalysator bei diesem Verfahren leicht regenerierbar sein.

Demgemäß wurden Verfahren zur Herstellung von Phenylacetaldehyden der allgemeinen Formel I

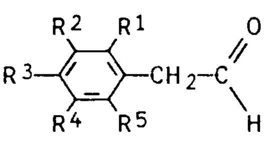
(I),

in der $R^1$ bis $R^5$ unabhängig voneinander Wasserstoff, Halogen, gegebenenfalls durch Halogen substituiertes Alkyl, Alkenyl, Alkoxyl, Alkylthio oder Cycloalkyl bedeuten, gefunden, welche dadurch gekennzeichnet sind, daß man Glycidsäureester der allgemeinen Formel II

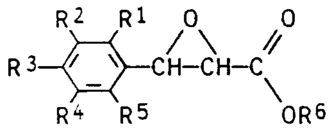

(II),

in der $R^1$ bis $R^5$ obige Bedeutung haben und $R^6$ für tert.-Butyl- oder i-Propyl steht, in Gegenwart von Zeolithen und/oder Phosphaten und/oder Phosphor- oder Borsäure auf Trägermaterial und/oder sauren Metalloxiden in der Flüssig- oder Gasphase bei Temperaturen von 50 bis 500 °C und Drücken von 0,01 bis 50 mbar umsetzt.

Durch die erfindungsgemäßen Verfahren wird den eingangs genannten Nachteilen der bislang bekannten Verfahren abgeholfen.

Die vorliegende Erfindung ermöglicht die Herstellung der Phenylacetaldehyde I aus gut zugänglichen Ausgangsstoffen in Gegenwart von Katalysatoren, die sich durch einfache Verfügbarkeit, hohe Aktivität und leichte Regenerierbarkeit auszeichnen. Weiterhin sind bei langen Katalysatorstandzeiten hohe Umsätze, hohe Selektivitäten und eine flexible Einsetzbarkeit des Katalysators hinsichtlich der Edukte gewährleistet.

Vorteile des erfindungsgemäßen Verfahrens an den erfindungsgemäßen Katalysatoren sind: vollständiger Umsatz, keine Trennprobleme, lange Standzeiten, Selektivitäten >90 %, auch sehr gute Ausbeuten bei halogenhaltigen Ausgangsprodukten, einfache Isolierung der Endprodukte, in der Regel Weiterverwendung ohne zusätzliche Reinigung, leichte Regenerierbarkeit der Katalysatoren bei eventuell auftretender Verkokung.

Die Phenylacetaldehyde I sind durch Umsetzung von Glycidsäureestern an Zeolithen bzw. anderen Katalysatoren erhältlich.

Nach dem erfindungsgemäßen Verfahren erfolgt die Umsetzung durch Kontakt eines Glycidsäureesters II mit Zeolithen und/oder Phosphaten und/oder Phosphor-oder Borsäure auf Trägermaterial und/oder sauren Metalloxiden in der Flüssig- oder Gasphase bei Temperaturen von 50 bis 500 °C und Drücken von 0,01 bis 50 bar nach folgendem Reaktionsschema:

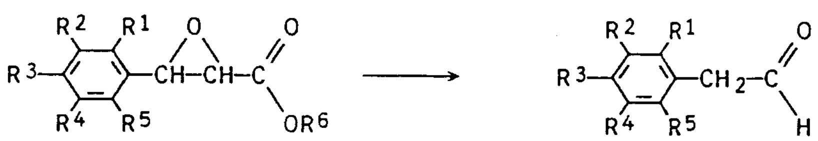

(II) (I)

Die Reaktion kann sowohl in der Flüssigphase als auch in der diskontinuierlichen oder vorzugsweise kontinuierlichen Gasphase bei 50 bis 500 °C und 0,01 bis 50 bar durchgeführt werden.

Die Flüssigphasenreaktion kann beispielsweise als Suspensions-, Riesel- oder Sumpfreaktion bei Temperaturen von 50 bis 200 °C und Drücken von 0,5 bis 20 bar, vorzugsweise bei Temperaturen von 70 bis 170 °C und Drücken von 1 bis 5 bar durchgeführt werden.

Die bevorzugte Gasphasenreaktion kann beispielsweise bei Temperaturen von 100 bis 500 °C, vorzugsweise bei 150 bis 400 °C und Drücken von 0,1 bis 50 bar, besonders bevorzugt bei 200 bis 350 °C und Drücken von 0,5 bis 5 bar, gegebenenfalls in Gegenwart von Inertgasen wie Stickstoff oder Argon, in einigen Fällen auch Sauerstoff oder auch z.B. Wasserdampf, durchgeführt werden. Bei der Umsetzung in der Gasphase hält man vorteilhaft eine Katalysatorbelastung von 0,1 bis 20, insbesondere 0,5 bis 5 g Glycidsäureester je g Katalysator und Stunde ein (WHSV = g Einsatzgemisch/g Katalysator und Stunde). Die Gasphasenreaktion kann in einem Festbett oder in einem Wirbelbett ausgeführt werden.

Die Reste $R^1$ bis $R^5$, die untereinander gleich oder verschieden sein können, bedeuten z.B. Methyl, Ethyl, n-/i-Propyl, n-/i-/t-Butyl, Hexyl, Octyl, Decyl, Dodecyl, Cyclopentyl, Cyclohexyl, Trifluormethyl, Trichlormethyl, Trifluormethoxy, Fluormethyl, Chlormethyl, Fluorcyclopentyl, Ethenyl, Propenyl, Butenyl, Hexenyl, Octenyl, Decenyl, Dodecenyl, Cyclopentenyl, Cyclohexenyl, Fluorcyclopentenyl, Trifluormethylthio, Fluor und Chlor.

Der Rest $R^6$ bedeutet tert.-Butyl und i-Propyl.

Als Katalysatoren für das erfindungsgemäße Verfahren werden acide zeolithische Katalysatoren eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome

verbunden sind. Das Verhältnis der Si- und Al-Atome zu Sauerstoff beträgt 1:2 (siehe Ullmanns Encyclopädie d. techn. Chemie, 4. Auflage, Band 24, Seite 575 (1983)). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt (siehe Ullmanns Encyclopädie d. techn. Chemie, 4. Auflage, Band. 24, S. 575 (1983)). So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben in "Catalysis by Zeolites", Band 5 aus "Studies in Surface Science and Catalysis" ed. B. Imelik et al. Elsevier Scientific Publishing Comp., 1980, 203 und "Crystal Structures of Ultra-stable Faujasites" Advances in Chemistry Series Nr. 101, American Chemical Society Washington, DC, S. 226 ff (1971) und in US-PS-4 512 961.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen (vgl. Ullmanns Encyclopädie d. techn. Chem., 4. Aufl., Bd. 24, 1983).

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220 °C unter autogenem Druck hergestellt. Auch gehören hierzu die isotaktischen Zeolithe nach EP-A-34 727 und EP-A-46 504. Die erhaltenen Aluminosilikatzeolithe enthalten je nach Wahl der Einsatzstoffmengen ein S $iO_2/Al_2O_3$-Verhältnis von 10 bis 40.000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200 °C unter autogenem Druck synthetisiert, indem man eine Borverbindung z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe nach EP-A-34 727 und EP-A-46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220 °C unter autogenem Druck.

Zu den verwendbaren siliciumreichen Zeolithen ($SiO_2/Al_2O_3 \geqq 10$) gehören auch die sog. ZSM-Typen, Ferrierit, Nu-1 und Silicalit®, ein Molekularsieb, ein sog. Silica Polymorph.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Alumino- bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalysatoren besteht z.B. darin, daß man den unverformten oder veformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. bis 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Fr, Yb und U eingesetzt.

Zweckmäßigerweise führt man die Dotierung so durch, daß man z.B. den verformten Zeolithen in einem Steigrohr vorlegt und bei 20 bis 100 °C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2$ x 3 $H_2O$ oder $Ni(NO_3)_2$ x 6 $H_2O$ oder $Ce(NO_3)_3$ x 6 $H_2O$ oder $La(NO_3)_2$ x 6 $H_2O$ oder $Cs_2CO_3$ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150 °C getrocknet und bei ca. 550 °C calciniert. Dieser Tränkvorgang kann mehrmals hintereinander vorgenommen werden, um den gewünschten Metallgehalt einzustellen.

Auch ist es möglich, z.B. eine wäßrige $Ni(CO_3)_2$-Lösung oder ammoniakalische $Pd(NO_3)_2$-Lösung herzustellen und darin den reinen pulverförmigen Zeolithen bei 40 bis 100 °C unter Rühren ca. 24 h aufzuschlämmen. Nach Abfiltrieren, Trocknen bei ca. 150 °C und Calcinierung bei ca. 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei ca. 150 °C getrocknet und bei ca. 550 °C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80 °C behandelt. Nach der Behandlung wird mit Wasser gewaschen, bei 110 °C/16 Stunden getrocknet und bei 500 °C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80 °C mit einer 3 bis 25 gew.%igen, insbesondere 12 bis 20 gew.%igen wäßrigen Salzsäure. Anschließend wird der so behandelte Zeolith mit Wasser gewaschen, getrocknet und bei 400 °C bis 500 °C calciniert.

Eine besondere Ausführungsform für die Säurebehandlung besteht darin, daß man das zeolithische Material vor seiner Formung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 bis 160 °C, getrocknet und bei Temperaturen von im allgemeinen 450 bis 600 °C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 bis 90 °C, vorzugsweise 60 bis 80 °C, über einen Zeitraum von 0,5 bis 5, vorzugsweise mit 12 bis 20 gew.%iger Salzsäure, behandelt. Anschließend wird das zeolithische Material im allgemeinen ausgewaschen und zweckmäßig, z.B. bei Temperaturen von 100 bis 160 °C, getrocknet und bei Temperaturen von im allgemeinen 450 bis 600 °C calciniert. Einer HF-Behandlung kann sich auch eine HCl-Behandlung anschließen.

Nach einer anderen Arbeitsweise lassen sich Zeolithe durch Aufbringung von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110 °C getrocknet und bei 500 °C calciniert.

Weitere Katalysatoren für das erfindungsgemäße Verfahren sind Phosphate, insbesondere Aluminiumphosphate, Siliciumaluminiumphosphate, Siliciumeisenaluminiumphosphate, Cerphosphat, Zirkonphosphate, Borphosphat, Eisenphosphat und deren Gemische.

Als Aluminiumphosphat-Katalysatoren werden für das erfindungsgemäße Verfahren insbesondere unter hydrothermalen Bedingungen synthetisierte Aluminiumphosphate eingesetzt, die Zeolithstruktur besitzen.

Die unter hydrothermalen Bedingungen hergestellten Aluminiumphosphate sind z.B. APO-5, APO-9, APO-11, APO-12, APO-14, APO-21, APO-25, APO-31 und APO-33. Synthesen dieser Verbindungen sind in EP 132 708, US 4 310 440 und US 4 473 663 beschrieben.

Beispielsweise das $AlPO_4$-5 (APO-5) wird synthetisiert, indem man Orthophosphorsäure mit Pseudoboehmit (Catapal SB®) in Wasser homogen mischt; zu dieser Mischung Tetrapropylammoniumhydroxid gibt und darach bei ca. 150 °C 20 bis 60 h unter autogenem Druck in einem Autoklaven umsetzt. Das abfiltrierte $AlPO_4$ wird getrocknet bei 100 bis 160 °C und calciniert bei 450 bis 550 °C.

$AlPO_4$-9 (APO-9) wird ebenfalls aus Orthophosphorsäure und Pseudoboehmit aber in wäßriger DABCO-Lösung (1,4-Diazabicyclo-(2,2,2)-octan) bei ca. 200 °C unter autogenem Druck während 200 bis 400 h synthetisiert.

Die Synthese des $AlPO_4$-21 (APO-21) erfolgt aus Orthophosphorsäure und Pseudoboehmit in wäßriger Pyrrolidon-Lösung bei 150 bis 200 °C und autogenem Druck während 50 bis 200 h.

Die für das erfindungsgemäße Verfahren eingesetzten Siliciumaluminiumphosphate sind z.B. SAPO-5, SAPO-11, SAPO-31 und SAPO-34. Die Synthese dieser Verbindung wird z.B. in EP 103 117, US 4 440 871 beschrieben. SAPO's werden hergestellt durch Kristallisation aus wäßriger Mischung bei 100 bis 250 °C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischung von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200 °C während 20 bis 200 h unter autogenem Druck in einem Rührautoklaven erhalten. Die Trocknung des abfiltrierten Pulvers erfolgt bei 110 bis 160 °C und die Calcination bei 450 bis 550 °C.

Als Phosphorkatalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g $Al(NO_3)_3$ x $H_2O$ in 700 ml Wasser über 2 h zugetropft. Dabei wird der pH-Wert durch gleichzeitige Zugabe von 25 %iger $NH_3$-Lösung bei pH 8 gehalten. Der entstandene Niederschlag wird 12 h nachgerührt, dann abgesaugt und ausgewaschen. Er wird bei 60 °C/16 h getrocknet.

Borphosphate für das erfindungsgemäße Verfahren lassen sich beispielsweise durch Mischen und Kneten von konzentrierter Borsäure und Phosphorsäure und durch anschließende Trocknung und Calcination in Inertgas-, Luft- oder Dampfatmosphäre bei 250 bis 650 °C, vorzugsweise 300 bis 550 °C, herstellen.

Auf diese Phosphate können durch Imprägnierung (Tränken und Aufsprühen) oder in manchen Fällen auch durch Ionenaustausch Modifizierungskomponenten, wie voran bei Zeolithen beschrieben, aufgebracht werden. Auch kann wie bei den Zeolithkatalysatoren eine Modifizierung mit Säuren erfolgen.

Geeignete saure Katalysatoren sind z.B. auch die sauer wirkenden Oxide von Elementen der III. und IV. Hauptgruppe sowie der IV. bis VI. Nebengruppe des periodischen Systems, insbesondere Oxide wie Siliciumdioxid in Form von Kieselgel, Kieselgur, Quarz, weiterhin Titandioxid, Zirkondioxid, Phosphoroxide, Vanadiumoxide, Nioboxide, Boroxide, Chromoxide, Molybdänoxide, Wolframoxide oder Bims oder Gemische dieser Oxide. Auch können diese Oxide durch Aufbringung von Modifizierungskomponenten, wie voran bei den Zeolithkatalysatoren beschrieben, dotiert werden. Die Behandlung mit Säuren, wie voran bei den Zeolithkatalysatoren beschrieben, ist ebenfalls eine eventuelle Möglichkeit der Modifizierung.

Auch mit Phosphorsäure oder Borsäure getränkte Katalysatoren können eingesetzt werden. Phosphorsäure oder Borsäure wird auf $SiO_2$-, $Al_2O_3$- oder Bims-Träger z.B. durch Auftränken oder Versprühen aufgebracht. Ein Phosphorsäure-haltiger Katalysator kann beispielsweise durch Auftränken von $H_3PO_4$- oder $NaH_2PO_4$- oder $Na_2HPO_4$-Lösung auf $SiO_2$ und anschließende Trocknung bzw. Calcination erhalten werden. Phosphorsäure kann aber auch mit Kieselgel zusammen in einem Sprühturm versprüht werden, danach schließt sich eine Trocknung und meist eine Calcination an. Phosphorsäure kann auch in einer Imprägniermühle auf das Trägermaterial aufgesprüht werden.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Nach dem erfindungsgemäßen Verfahren können schwerflüchtige oder feste Ausgangsstoffe in gelöster Form z.B. in THF-, Toluol- oder Petrolether-Lösung eingesetzt werden. Auch eine Verdünnung mit den genannten Lösungsmitteln ist möglich, wobei pro Mol II zwischen 100 und 500 ml, vorzugsweise 150 bis 350 ml eines dieser Lösungsmittel in aller Regel genügt.

Nach der Umsetzung werden die Phenylacetaldehyde nach üblichen Verfahren z.B. durch Destillation aus dem Reaktionsgemisch isoliert; nicht umgesetzte Ausgangsstoffe gegebenenfalls in die Umsetzung zurückgeführt. Auch eine direkte Weiterverarbeitung der Reaktionsprodukte ist aufgrund der sehr hohen Ausbeuten möglich. Bei dem Verfahren fallen bevorzugt die monomeren Verbindungen an. Sollten auch Oligomere z.B. trimere Phenylacetaldehyde gebildet werden, so können diese abgetrennt und zu den gewünschten Monomeren nach bekannten Methoden gespalten werden.

Die nach dem erfindungsgemäßen Verfahren zugänglichen Verbindungen sind wichtige Zwischenprodukte für biologisch aktive Verbindungen, z.B. Insektizide (Resmethrin), Fungiziden, Herbiziden und im Pharmabereich. Weiterhin kann man sie nach geläufigen Methoden, z.B. durch Oxidaton mit Sauerstoff oder durch Reduktion z.B. durch katalytische Hydrierung oder hydrierende Aminierung zu Aminen, Alkoholen und Säuren weiterverarbeiten, die ihrerseits wertvolle Zwischenprodukte sind.

Die folgenden Beispiele veranschaulichen die Erfindung:

Die für das erfindungsgemäße Verfahren eingesetzten Katalysatoren sind:

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 Stunden getrocknet und bei 500 °C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit einem Verformungshilfsmittel 2-mm-Stränge hergestellt, die bei 110 °C/16 h getrocknet und bei 500 °C/24 h cacliniert werden.

Katalysator B

Ein Aluminosilikatzeolith vom Pentasil-Typ wurde unter hydrothermalen Bedingungen bei autogenem Druck und 150 °C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50:50 Gew.%) in einem Rührautoklaven hergestellt. Nach Abfiltrieren und Auswaschen wurde das kristalline Reaktionsprodukt bei 110 °C/24 Stunden getrocknet und bei 500 °C/24 Stunden calciniert. Dieser Aluminosilikatzeolith enthielt 91,6 Gew.% $SiO_2$ und 4,6 Gew.% $Al_2O_3$.

Der Katalysator wurde mit einem Verformungshilfsmittel zu 2-mm-Strängen verformt, bei 110 °C/16 Stunden getrocknet und bei 500 °C/24 Stunden calciniert.

Katalysator C

Katalysator C wird erhalten, indem man die Stränge des Katalysators A mit einer wäßrigen $Cs_2CO_3$-Lösung imprägniert, danach bei 130 °C/2 h trocknet und bei 540 °C/2 h calciniert. Der Cs-Gehalt beträgt 0,6 Gew.%.

Katalysator D

Der Eisensikikatzeolith des Pentasil-Typs wurde unter hydrothermalen Bedingungen bei autogenem Druck und 165 °C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Schwefelsäure und 425 g Wasser in einem Rührautoklaven während 4 Tagen synthetisiert. Der Zeolith wurde abfiltriert, ausgewaschen, bei 100 °C/24 Stunden getrocknet und bei 500 °C/24 h calciniert. Man erhielt einen Eisensilikatzeolithen mit einem $SiO_2/Fe_2O_3$-Verhältnis von 17,7 und einen $Na_2O$-Gehalt von 1,2 Gew.%. Der Katalysator wurde hochdispersem $SiO_2$ im Gewichtsverhältnis 70:30 zu 2,5-mm-Strängen verstrangt, bei 110 °C/16 Stunden getrocknet und bei 500 °C/24 Stunden calciniert. Diese Stränge werden mit einer 20 %igen $NH_4Cl$-Lösung bei 80 °C ionenausgetauscht und danach chloridfrei gewaschen, bei 110 °C getrocknet und bei 500 °C/5 h calciniert. Der Ionenaustausch wird so lange vorgenommen, bis der Na-Gehalt 0,002 Gew.% beträgt.

Katalysator E

Der Katalysator E wird wie Katalysator C hergestellt, wobei jedoch $Cs_2CO_3$ durch $Ce(NO_3)_2$ ersetzt wird. Der Ce-Gehalt beträgt 1,8 Gew.% .

Katalysator F

Siliciumaluminiumphosphat-5 (SAPO-5) wird hergestellt aus einer Mischung von 200 g 98 %iger Phosphorsäure, 136 g Boehmit, 60 g Kieselöl (30 %ig), 287 g Tripropylamin und 587 g $H_2O$. Diese Mischung wird bei 150 °C während 168 h unter autogenem Druck umgesetzt. Nach Filtration wird das kristalline Produkt bei 120 °C getrocknet und bei 500 °C calciniert. SAPO-5 enthält 49,8 Gew.% $P_2O_5$, 33,0 Gew.% $Al_2O_3$, 6,2 Gew.% $SiO_2$. SAPO-5 wird mit einem Verstrangungshilfsmittel zu 3-mm-Strängen verformt, bei 120 °C getrocknet und bei 500 °C calciniert.

Katalysator G

Kommerziell erhältliches Zirkonphosphat $Zr_3(PO_4)_4$ in Reinsubstanz verformt.

Katalysator H

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3-mm-Strängen verformt. Diese Stränge werden bei 100 °C getrocknet und bei 350 °C calciniert. Katalysator H enthält 8,77 Gew.% B und 28,3 Gew.% P.

Katalysator I

$TiO_2$ P 25® wird zu 2-mm-Strängen verformt, bei 110 °C getrocknet und bei 500 °C/16 h calciniert.

Katalysator J

D 10-10® $Al_2O_3$ wird mit $H_3BO_3$ imprägniert, getrocknet bei 110 °C und bei 500 °C/5 h calciniert. Der Katalysator K setzt sich zusammen aus 85 % $Al_2O_3$ und 15 % $B_2O_3$.

Die mit diesen Katalysatoren erzielten Versuchsergebnisse und Versuchsbedingungen sind in Tabelle 3 + 4 zusammengefaßt.

Beispiele 1 bis 15

Die Reaktionen in der Gasphase wurden unter isothermen Bedingungen in einem Rohrreaktor (Wendel, 0,6 cm Innendurchmesser, 90 cm Länge) mindestens 6 Stunden lang durchgeführt. Die Reaktionsprodukte wurden durch übliche Methoden abgetrennt und charakterisiert. Die quantitative Bestimmung der Reaktionsprodukte und der Ausgangsprodukte erfolgte gaschromatographisch.

Die Ergebnisse sind in den Tabellen 1 und 2 zusammengestellt.

Tabelle 1: Umsetzung von p-Methylphenylglycidsäure-tert.-butylester zu p-Methylphenylacetaldehyd

| Beispiel | 1 | 2 | 3 | 4 | 5 | 6 | 7 | 8 | 9 | 10 | 11 |
|---|---|---|---|---|---|---|---|---|---|---|---|
| Katalysator | A | A | B | C | D | E | F | G | H | I | J |
| Temp., °C | 350 | 300 | 250 | 250 | 250 | 250 | 300 | 250 | 250 | 300 | 300 |
| WHSV $h^{-1}$ | 3 | 3 | 3 | 3 | 3 | 3 | 3 | 3 | 3 | 3 | 3 |
| Umsatz % | 100 | 100 | 100 | 100 | 100 | 100 | 100 | 100 | 100 | 100 | 100 |
| Selekt. % | 93,5 | 89,2 | 87,2 | 94,0 | 87,9 | 89,9 | 87,1 | 80,1 | 85,7 | 78,9 | 83,7 |

Tabelle 2: Umsetzung von substituierten Phenylglycidsäure-tert.-butylester zu den entsprechenden Phenylacetaldehyden

| Beispiel | 12 | 13 | 14 | 15 |
|---|---|---|---|---|
| Substituent | p-Fluor | p-Methoxy | p-Trifluor-methyl | 1-Methyl-4-fluor |
| Katalysator | A | A | A | A |
| Temperatur °C | 250 | 250 | 250 | 250 |
| $WHSV^{-1}$ | 3 | 3 | 3 | 3 |
| Umsatz % | 100 | 100 | 100 | 100 |
| Selektivität % | 91,9 | 90,1 | 93,9 | 88,7 |

Beispiel 16

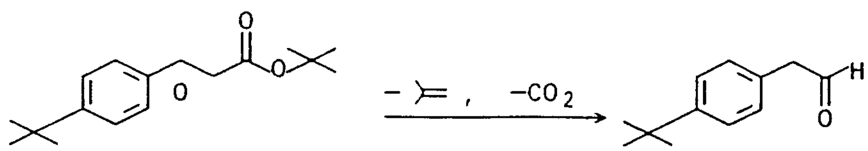


100 g p-tert.-Butylphenylglycidsäure-tert.-butylester werden pro Stunde im Gleichstrom mit 400 Nl Stickstoff pro Stunde über einen auf 260 °C erhitzten Borzeolithkatalysator A geleitet, der sich in einem elektrisch beheizten 1-l-Rohrreaktor befindet. Die den Reaktor verlassenden Reaktionsprodukte werden kondensiert und destillativ aufgearbeitet. p-tert.-Butylphenylacetaldehyd (Kp = 87 °C/0,4 mbar) wird dabei in einer Ausbeute von 92 % d.Th. erhalten.

## Patentansprüche

1. Verfahren zur Herstellung von Phenylacetaldehyden der allgemeinen Formel I

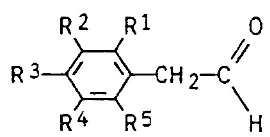 (I),

in der $R^1$ bis $R^5$ unabhängig voneinander Wasserstoff, Halogen, gegebenenfalls durch Halogen substituiertes Alkyl, Alkenyl, Alkoxyl, Alkylthio oder Cycloalkyl bedeuten, dadurch gekennzeichnet, daß man Glycidsäureester der allgemeinen Formel II

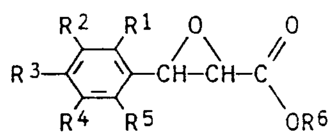 (II),

in der $R^1$ bis $R^5$ obige Bedeutung haben und $R^6$ für tert.-Butyl oder i-Propyl steht, in Gegenwart von Zeolithen und/oder Phosphaten und/oder Phosphor- oder Borsäure auf Trägermaterial und/oder sauren Metalloxiden in der Flüssig- oder Gasphase bei Temperaturen von 50 bis 500 °C und Drücken von 0,01 bis 50 bar umsetzt.

2. Verfahren nach Anspruch 1, dadurch gekennzeichnet, daß man als Katalysatoren Zeolithe des Pentasil- oder Faujasit-Typs verwendet.

3. Verfahren nach Anspruch 1, dadurch gekennzeichnet, daß man als Katalysatoren Aluminiumsilikatzeolithe, Borosilikatzeolithe oder Eisensilikatzeolithe des Pentasiltyps verwendet.

4. Verfahren nach Anspruch 1, dadurch gekennzeichnet, daß man mit Alkalimetallen, Übergangsmetallen, Seltenen Erdmetallen dotierte Zeolithe als Katalysatoren verwendet.

5. Verfahren nach Anspruch 1, dadurch gekennzeichnet, daß man als Katalysatoren Phosphate der Elemente B, Al, Zr, Ce, Fe, Sr oder deren Gemische oder hydrothermal hergestellte Phosphate verwendet.

6. Verfahren nach Anspruch 1, dadurch gekennzeichnet, daß man als Katalysatoren hydrothermal hergestellte Aluminiumphosphate oder Siliciumaluminiumphosphate oder Siliciumeisenaluminiumphosphate oder Boraluminiumphosphate verwendet.

7. Verfahren nach Anspruch 1, dadurch gekennzeichnet, daß man als Katalysatoren saure Metalloxide der Elemente Ti, Zr, Al, Si, V, W, Nb, Cr oder Phosphorsäure bzw. Borsäure auf Trägermaterialen $SiO_2$, $Al_2O_3$, $TiO_2$ und Bims verwendet.

8. Verfahren nach Anspruch 1, dadurch gekennzeichnet, daß man die Reaktion in der Gasphase durchführt.

## Claims

1. A process for preparing phenylacetaldehydes of the general formula I

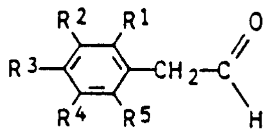

(I),

where $R^1$ to $R^5$ are independently of one another hydrogen, halogen, unsubstituted or halogen-substituted alkyl, alkenyl, alkoxyl, alkylthio or cycloalkyl, which comprises reacting glycidic esters of the general formula II

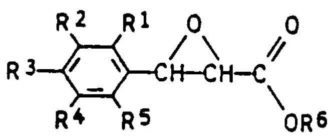

(II),

where $R^1$ to $R^5$ are each as defined above and $R^6$ is tert-butyl or isopropyl, in the liquid or gas phase at from 50 to 500 °C and at from 0.01 to 50 bar in the presence of zeolites and/or phosphates and/or phosphoric or boric acid on a carrier material and/or acidic metal oxides.

2. A process as claimed in claim 1, wherein the catalyst used is a zeolite of the pentasil or faujasite type.

3. A process as claimed in claim 1, wherein the catalyst used is an aluminum silicate zeolite, a borosilicate zeolite or an iron silicate zeolite of pentasil type.

4. A process as claimed in claim 1, wherein the catalyst used is a zeolite doped with an alkali metal, a transition metal or a rare earth metal.

5. A process as claimed in claim 1, wherein the catalyst used is a phosphate of the element B, Al, Zr, Ce, Fe or Sr or of a mixture thereof or a hydrothermally synthesized phosphate.

6. A process as claimed in claim 1, wherein the catalyst used is a hydrothermally synthesized aluminum phosphate or silicon aluminum phosphate or silicon iron aluminum phosphate or boron aluminum phosphate.

7. A process as claimed in claim 1, wherein the catalyst used is an acidic metal oxide of the element Ti, Zr, Al, Si, V, W, Nb or Cr, or phosphoric or boric acid on $SiO_2$, $Al_2O_3$, $TiO_2$ or pumice.

8. A process as claimed in claim 1, wherein the reaction is carried out in the gas phase.

## Revendications

**1.** Procédé de préparation de phénylacétaldéhydes de formule générale I

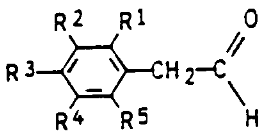

(I),

dans laquelle $R^1$ à $R^5$ représentent, indépendamment les uns des autres, des atomes d'hydrogène ou des restes alkyle, alcényle, alcoxy, alkylthio ou cycloalkyle, caractérisé en ce qu'on fait réagir des esters d'acide glycidique de formule générale II

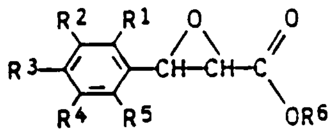

(II),

dans laquelle $R^1$ à $R^5$ ont les significations susmentionnées et $R^6$ est mis pour un reste tert.-butyle ou i-propyle, en présence de zéolithes et/ou de phosphates et/ou d'acide phosphorique ou borique sur une matière de support et/ou d'oxydes métalliques acides, en phase liquide ou gazeuse à des températures de 50 à 500 °C et sous des pressions de 0.01 à 50 bar.

**2.** Procédé selon la revendication 1, caractérisé en ce qu'on utilise, comme catalyseurs, des zéolithes du type pentasil ou faujasite.

**3.** Procédé selon la revendication 1, caractérisé en ce qu'on utilise, comme catalyseurs, des zéolithes d'aluminosilicate, des zéolithes de borosilicate ou des zéolithes de ferrosilicate du type pentasil.

**4.** Procédé selon la revendication 1, caractérisé en ce qu'on utilise, comme catalyseurs, des zéolithes dopées avec des métaux alcalins, des métaux de transition ou des métaux des terres rares.

**5.** Procédé selon la revendication 1, caractérisé en ce qu'on utilise, comme catalyseurs, des phosphates des éléments B, Al, Zr, Ce, Fe, Sr ou de mélanges de ceux-ci ou des phosphates préparés par synthèse hydrothermale.

**6.** Procédé selon la revendication 1, caractérisé en ce qu'on utilise, comme catalyseurs, des phosphates d'aluminium, des phosphates de silicium et d'aluminium, des phosphates de silicium de fer et d'aluminium ou des phosphates de bore et d'aluminium, préparés par synthèse hydrothermale.

**7.** Procédé selon la revendication 1, caractérisé en ce qu'on utilise, comme catalyseurs, des oxydes métalliques acides des éléments Ti, Zr, Al, Si, V, W, Nb, Cr ou de l'acide phosphorique ou borique sur les matières de support $SiO_2$, $Al_2O_3$, $TiO_2$ ou pierre ponce.

**8.** Procédé selon la revendication 1, caractérisé en ce qu'on conduit la réaction en phase gazeuse.